# EUROPEAN PATENT APPLICATION

(11) **EP 0 774 329 A1**
(43) Date of publication of application: **21.05.1997**
(21) Application number: 96308401.7
(22) Date of filing: 20.11.1996
(51) Int. Cl.: B25J 3/04, B25J 13/02, A61B 19/00

(54) **Telerobotic laparoscopic manipulator**

(30) Priority: 20.11.1995 US 560611
(71) Applicant: ETHICON ENDO-SURGERY, INC., Cincinnati, Ohio 45242 (US)
(72) Inventor: Stubbs, Jack, Waynesville, Ohio 45068 (US); Bohanan, Bryan, Hamilton, Ohio 45011 (US)
(74) Representative: Fisher, Adrian John

(57) **Abstract**

A manipulable hand for use in laparoscopic surgery having a controlled hand (12), remote from the operator, and having at least one controlled finger (72,76). The controlled finger has at least one hinge corresponding to an interphalangeal joint of a human finger and is adapted for insertion through a cannula to an internal surgical site. The controlled hand is connected by motion transmission rods (50-55) or cables (50a-54a) to a controller (14) which moves the controlled hand in accordance with sensed movement of the surgeon's hand and allows a surgeon to remotely perform a laparoscopic surgical procedure.

## Description

### BACKGROUND OF THE INVENTION

This invention relates to surgical instruments and, more particularly, to a manipulable hand for use in laparoscopic surgery which is remotely controlled by sensed movement of a surgeon's hand.

Laparoscopic surgical techniques involve performing surgical procedures through a small diameter cannula. These techniques minimize trauma to surrounding tissue and organs and greatly reduce the patient's recovery period. To perform these laparoscopic techniques, a small incision is made, and a small cannula is inserted through the incision. The cannula defines a passageway through which various instruments can be inserted to perform procedures, such as cutting, suturing and removal of tissue.

Laparoscopic surgical techniques have essentially removed the surgeon's hands from the patient's body and replaced them with blades, suture needles, and small graspers. In order to achieve the goals of laparoscopic surgery, means provided as a substitute for the surgeon's hands should be as similar as possible to the real thing. The closest approximations presently in use are grasping devices of rather approximate design, and devices capable only of cutting, scraping, or similar actions. All of these are somewhat limited compared to the human hand wielding an appropriate surgical tool.

Known laparoscopic techniques allow the surgeon only limited capabilities within the surgical site. Known laparoscopic techniques also require the surgeon be present in the operating room with the patient. While this is not always undesirable, it would be more efficient if the surgeon could perform the laparoscopic surgery from a location remote from the patient and the operating room, thereby allowing the most skilled surgeons to perform any given surgery regardless of the location of the patient.

Therefore, there exists a need in the art for a device which will enable the surgeon to perform remote laparoscopic surgery, and which will mimic movement of the surgeon's hand to allow the performance of "virtual" surgery.

### SUMMARY OF THE INVENTION

The present invention overcomes the problem arising from the conflicting need for obtaining the benefits of skilled, delicate control of surgical procedures while simultaneously achieving the benefits of laparoscopic surgical techniques.

According to the preferred embodiment of the present invention, a manipulable controlled hand for laparoscopic surgery has three controlled digits, the digits comprising a thumb and two fingers. The controlled digits are movably attached to a mounting base positioned upon the distal end of a shaft. The controlled digits, the mounting base and a portion of the shaft are insertable through a cannula to an internal surgical site.

In further accordance with the present invention, a proximal end of the shaft is adjacent a control means which controls operation and movement of the controlled hand digits and wrist in response to the sensed position and movement of the corresponding wrist and digits of the surgeon's hand. Each of the controlled digits is connected and responsive to a homologous digit on the surgeon's hand. The controlled hand comprises hinged sections corresponding and responsive to those of the surgeon's hand. Thus, the articulations of the controlled hand correspond to the articulations of the surgeon's hand, allowing the controlled hand to duplicate the actions and responses of the surgeon's hand as closely as possible.

The controlled digits are capable of motion independent of one another, and each is capable of flexion and extension. The controlled fingers are capable of spreading apart, i.e., moving horizontally away from and back toward each other, between a first substantially aligned position and a second, angularly related V-shaped position. The controlled thumb is opposable, and is capable of spreading away from and moving toward the fingers, in motions known as palmar abduction and adduction, respectively.

According to the present invention, movement of the controlled hand is directed and mechanically controlled by the controller, and the operative connections between the controller and the controlled hand comprise control rods and/or cables. The controller can scale movement of the controlled hand to any fraction or multiple of the sensed movement of the surgeon's hand, as would be desirable in numerous surgical applications (i.e., microsurgery).

The manipulable hand of the present invention is intended to be used with other known laparoscopic techniques. Of particular importance are visualization techniques by means of which the surgeon remotely views the surgical site. The usual visualization techniques include the use of fiber optic illumination and video display, but may include other less direct methods such as fluorometry, X-ray, magnetic resonance imaging and computerized axial tomography.

One method of providing illumination for visualization is described in the commonly assigned application, European Patent Application No. 93302849.0, filed April 13, 1993, which application is hereby incorporated herein by reference. That application describes an illuminated surgical cannula, comprising optical fibers embedded within the fiber composite body of the cannula. In that invention, illumination is externally provided to optical fibers integral to the body of the cannula. The integral fibers transmit the light into the incision and the surgical site. Illumination thus provided allows the operator to visualize the site by known laparoscopic methods, such as video transmission.

### BRIEF DESCRIPTION OF THE DRAWINGS

These and further features of the present invention will be apparent with reference to the following description and drawings, wherein:
Fig. 1 is a schematic diagram of the surgical system according to the present invention;
Fig. 2 is a perspective view of a controlled hand and sleeve according to the present invention;

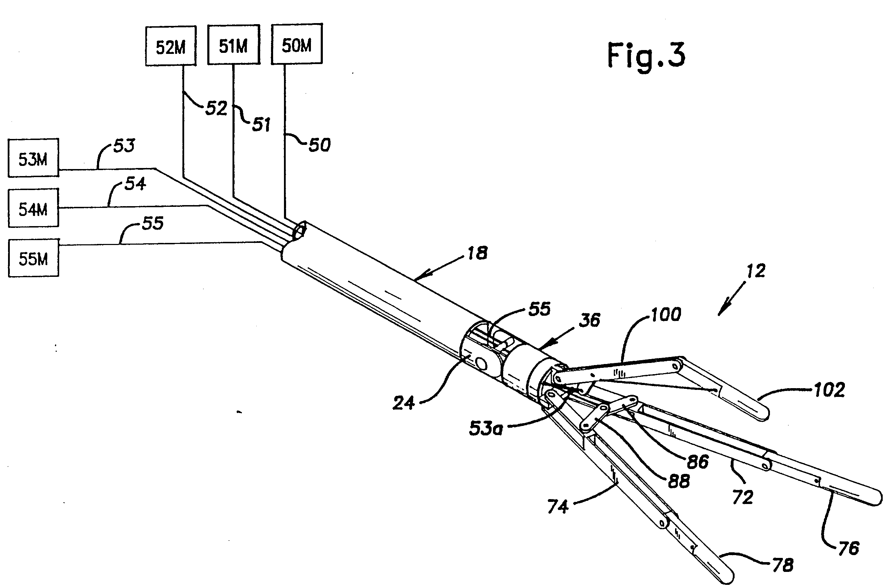

Fig. 3 is a perspective view of the hand and sleeve, with the controlled digits in a different position;

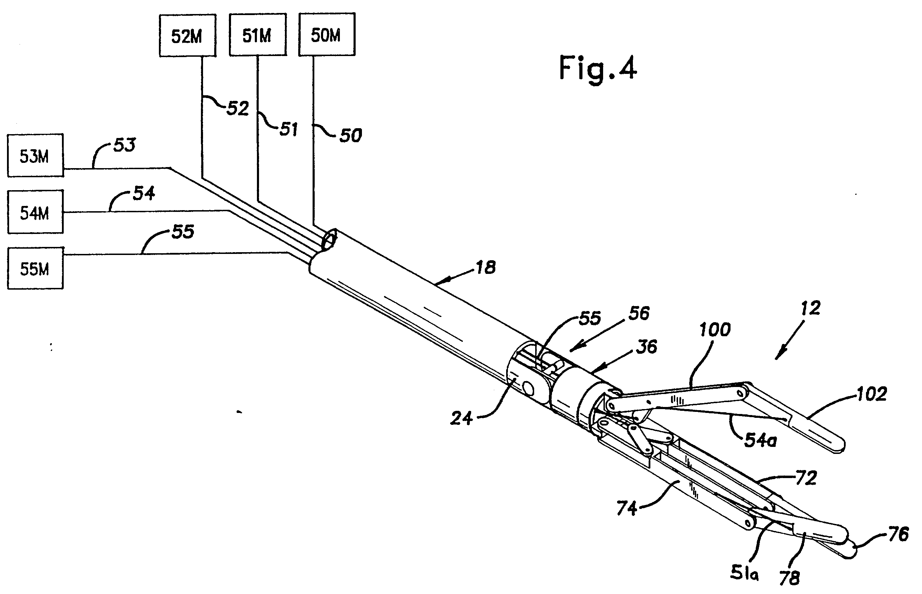

Fig. 4 is a perspective view similar to Fig. 3, but with the digits in another displaced position;

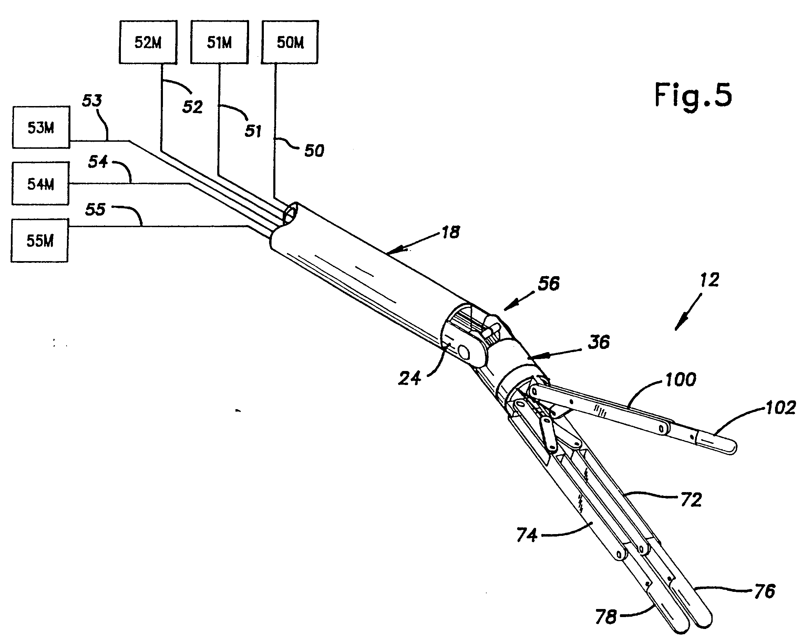

Fig. 5 is a perspective view similar to Fig. 2, but showing the controlled wrist in a displaced position; and
Fig. 6 is an exploded perspective view of the controlled hand according to the present invention.

### DETAILED DESCRIPTION OF THE PREFERRED EMBODIMENT

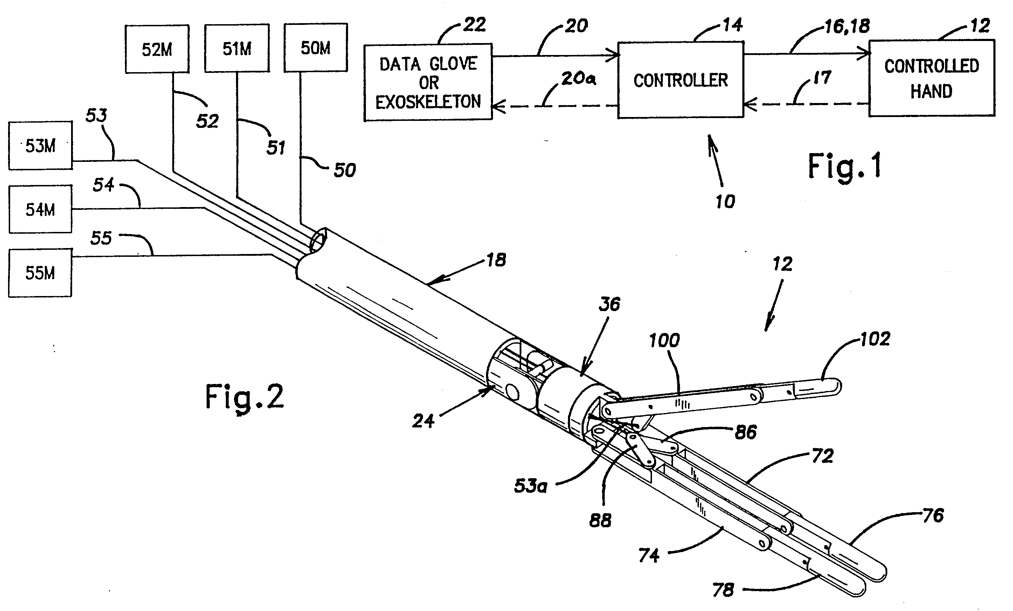

With reference to Fig. 1, a telerobotic manipulator 10 according to the present invention includes a distal or controlled hand 12, a controller 14 for controlling the controlled hand, and a "data glove" or exoskeleton 22. The controller comprises a microprocessor which uses signals developed by the data glove or exoskeleton 22 to control actuation of the servomotors, as will be discussed hereafter. A motion transmitting device 16 extends between the controller 14 and the controlled hand, and includes a hollow, elongated tube 18 through which a series of motion transmission rods extend. A data feedback 17 may extend from the controlled hand 12 to the controller 14 and be operable to transmit signals indicative of pressure sensed by sensors (not shown) on the controlled hand 12.

As will be discussed more fully hereafter, the motion transmission rods are each connected, at a proximal end, to a corresponding servomotor and, at a distal end, to a portion of the controlled hand. The rods are operable, due to actuation of associated servomotors by the microprocessor, to move the controlled hand in a predictable and desired fashion.

A data link 20 extends between the data glove or exoskeleton 22 and the controller 14. The data link 20 may be an electrical or fiber optic cable when the surgeon is physically present in the operating room. Alternatively, the data link may be a telephone line and/or satellite connection if the surgeon is located remotely from the operating room. A similar data feedback link 20a may optionally be provided to transmit feedback signals to the data glove or exoskeleton indicative of pressure experienced at the controlled hand 12.

Remote visualization of the surgical field is from the controller 14, preferably provided by standard laparoscopic surgical visualization techniques. Visualization can be provided to a remote surgeon via a dedicated phone line or satellite link.

Movement and positioning of the relevant fingers or digits of the surgeon's hand are sensed by the data glove or exoskeleton 22. A data glove is a glove-like device which is worn by an operator and includes a plurality of sensors to sense the relative position and movement of the operator's fingers and wrist. Similarly, an exoskeleton provides a framework which is mounted over the operator's hand and includes sensors to sense the relative position and movement of the operator's fingers and wrist. The data glove and exoskeleton are considered generally interchangeable in the present application. One data glove which has been successfully used by the present inventors is sold under the tradename "CYBERGLOVE" by Virtual Technologies of Palo Alto, California. The sensed position signals developed by the data glove or exoskeleton 22 are transmitted through the data link 20 to the controller and are used by the controller to control movement of the controlled hand 12, as will be discussed more fully hereafter. The data glove or exoskeleton 22 may optionally include transducers to transform feedback signals from the data feedback link 20a into pressure changes that can be felt by the surgeon's hand.

The following describes the structure and operation of the illustrated embodiment of the controlled hand 12 and the controlled wrist 56, which is shown best in Fig. 7. The distal end of the tube 18 is attached to a cylindrical portion of a distal support post 24. Preferably the inner diameter of the cylindrical portion of the support post 24 is selected to snugly receive tube 18 therein. The support arms 26, 28 have coaxial circular openings 30, 32 which receive a pivot pin 34 that pivotally attaches the support post 24 to a mounting base pivot post 36.

The pivot post 36 has two pivot arms 38, 40 pivotally attached to the support post arms 26, 28. The pivot arms 38, 40 have coaxial circular openings 42, 44, as illustrated. The pivot arms 38, 40 fit snugly but pivotally between the support arms 26, 28, and the openings 30, 32, 42, and 44 are aligned by the pivot pin 34. The attachment allows free swiveling movement of the pivot post 36 about a generally horizontal axis which is transverse to the length direction of the pivot post 36 and support post 24, thereby allowing the joint to act as a wrist.

The six motion transmission rods 50, 51, 52, 53, 54, and 55 extend from the controller 14, through the elongated tube 18, and emerge through a central opening 25 defined by the support post 24. Stiffly flexible cables 50a, 51a, 52a, 53a, 54a are each attached to a corresponding control rod 50, 51, 52, 53, 54 and continue into and through the cylindrical portion of the pivot post 36, to control operation of the controlled hand 12.

The sixth motion transmission rod 55 is connected at one end to its associated servomotor 55M, passes through the hollow portions of the tube 18 and the support post 24, and is pivotally connected to a wrist actuating pin 60 in the controlled wrist 56. The wrist actuating pin 60 is received within and extends between coaxial pivot-actuating openings 62, 64 provided in the pivot arms 38, 40. The pivot actuating openings 62, 64 are slightly vertically offset from the pivot arm openings 42, 44, such that forces transmitted to the wrist actuating pin 60 via the sixth motion transmission rod 55 causes the pivot post 36 to travel or orbit about the pivot pin 34. As such, the pivot pin 34 acts as a fulcrum or axis about which the controlled wrist 56 bends.

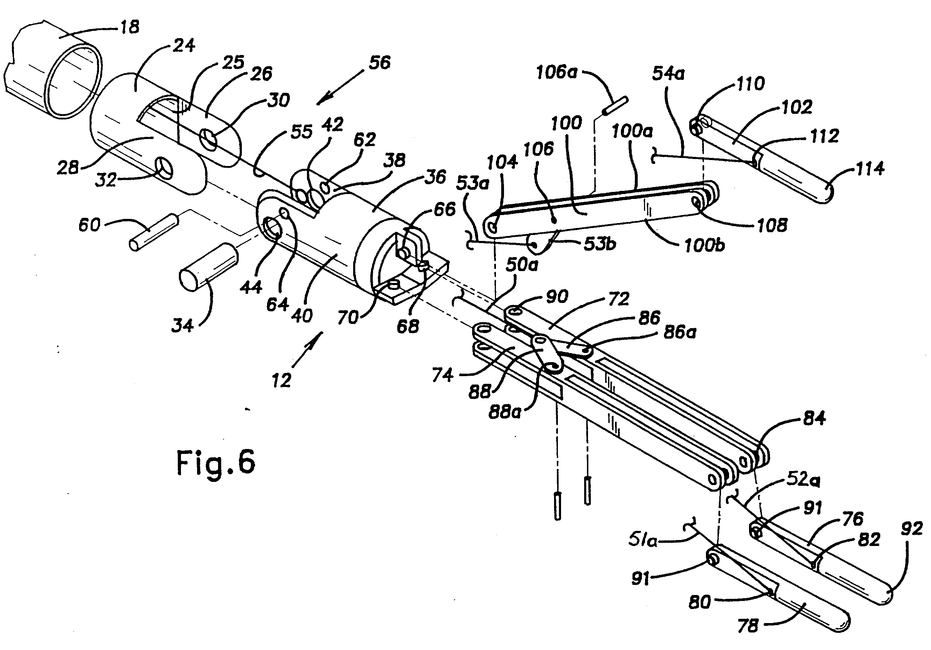

The pivot post 36 has a distal end which includes mounting positions for each of the digits of the controlled hand 12. As shown in Fig. 6, in the preferred embodiment mountings are provided for three digits, a thumb and two fingers. Thumb mounting studs 66 (one shown) provide horizontally oriented posts allowing vertical pivoting of the controlled thumb about an axis defined by the studs 66. Pivotal movement of the controlled thumb corresponds to palmar adduction and abduction of the surgeon's thumb.

Two pair of finger mounting studs 68 and 70 (one of each shown in Fig. 6) are vertically oriented posts allowing the controlled fingers to pivot about a generally vertical axis, which corresponds to a spreading movement of the surgeon's finger, as will be discussed hereafter.

The first phalanges 72, 74 of the controlled fingers are directly attached to the finger mounting studs 68, 70, correspond to the first phalange of the surgeon's finger, and are referred to as a controlled left finger first phalange 72 and a controlled right finger first phalange 74. Each first phalange 72, 74 has a second phalange 76, 78 attached thereto. The point of attachment between the first and second phalanges is an interphalangeal joint hinge. The interphalangeal joint hinge at which the first and second phalanges are attached to one another corresponds to an interphalangeal joint on the surgeon's finger. In operation, these correspondences are one-for-one.

It should be noted that the term "one-for-one" correspondence as used herein does not necessarily mean that the movements of the corresponding elements are exactly of the same magnitude or in exactly the same direction. Rather, "one-for-one" correspondence means that for each position of a particular element, there is one and only one position of the corresponding element. Generally, the positions of the elements of the controlled hand have a one-for-one correspondence with the surgeon's hand, but may not have the same magnitude of motion.

Each second phalange 76, 78 is actuated by a motion transmission cable 5la, 52a which extend from cable mounts 80 and 82, respectively, on the second phalanges 78 and 76 to the end of the second and third motion transmission rods 51 and 52, respectively. Thus, the controlled fingers are capable of moving in flexion and extension in one-for-one correspondence with the surgeon's fingers.

In operation, as will be described more fully hereafter, each second phalange of the surgeon's fingers is operably or functionally linked to the corresponding second phalange 76, 78 of the controlled hand 12 by the data link 20, controller 14 (i.e., microprocessor and servomotors), flexible cables and motion transmission rods. One end of the motion transmission rods 51, 52 is connected to its corresponding second phalange 76, 78 by stiffly flexible cables 51a, 52a, which, along with the motion transmitting rods, constitute a portion of the motion transmitting device 16. The opposite end of the motion transmission rods 51, 52 is connected to the corresponding servomotor 51M, 52M.

The left and right controlled fingers are identical to each other in the preferred embodiment depicted in Fig. 6, and so only the left controlled finger will be specifically described, except to the extent necessary to describe the interactions of the left and right controlled fingers.

The left first phalange 72 preferably provides channels to retain the motion transmission cables and rods out of the way while being in a position to provide the maximum level of control to the surgeon. Thus, the first phalanges 72, 74 may be tubular or channeled. As shown in Fig. 6, the first phalange preferably has a rearward, horizontally-oriented channel segment and a forward, vertically-oriented channel segment.

The horizontally-oriented channel segment allows for horizontal mounting of the finger and swiveling movement of the finger in a horizontal plane (i.e., about a vertical axis). The horizontal channel allows a laterally-oriented connection between the first motion transmitting rod 50 and the controlled fingers, the motion transmission rod 50 being disposed between the controlled fingers and causing the fingers to spread outwardly in the same plane.

The vertically oriented channel segment preferably provides a passageway for the flexible cables 5la, 52a used to actuate movement of the second phalange 76, 78 attached to each first phalange 72, 74. This segment may be made from material either the same or different, tubular or channeled, as the horizontal segment. At the distal end of this segment are a pair of coaxial openings to facilitate mounting of the second phalange 76 to the first phalange 72. As depicted in Fig. 6, the second phalange 76 is thereby permitted to swivel or move in a vertical plane (i.e., about a horizontal axis), co-planar with the first phalange.

Spreader links 86, 88 are located near the union of the horizontal and vertical channel segments provided by the first phalanges 72, 74. The distance of the spreader links from the base mount 90 may be varied to alter various operational parameters, such as the maximum spreading angle between the controlled fingers, the ratio of distally linear movement of the first motion transmission rod 50 to the angle or distance between the spread fingers, and the length of the motion transmission rod 50 exposed during spreading.

The relative length of the horizontally and vertically oriented channel segments of first phalange 72, 74 is variable within a wide range. Preferably, the channel segments should be sized such that the first phalanges 72, 74 are freely horizontally movable by operation of the spreading links 86, 88 attaching the first phalange to the first motion transmission rod 50, and the second phalange 76, 78 should be freely movable through its designed range of motion in response to movement of the associated motion transmission rod 51, 52.

The second phalange 76 includes a pair of mounting posts 91 on its proximal end that fit into the coaxial openings 182 in the first phalange 72. The second phalange 76 may preferably be made from round stock, with a flattened segment extending part of the length of the second phalange. The flattened segment will preferably extend from the proximal end of the second phalange at least to the vicinity of a flexion cable mount opening 82, as illustrated. A tip 92 of the second phalange 76 is preferably cylindrical, with a rounded distal end.

The second phalange 76 is actuated and controlled by the flexible cable 52a connected to the distal end of the third motion transmission rod 52, which is in turn actuated and controlled by its associated servomotor 52M. The servomotor 52M is operated by the microprocessor provided by the controller 14 in response to sensed movement of the second phalange of the surgeon's left finger.

Similarly, the second phalange 78 of the right controlled finger is actuated and controlled by the flexible cable 5la connected to the distal end of the second motion transmission rod 51. The second motion transmission rod 51 is actuated and controlled by its associated servomotor 51M. The servomotor 51M is operated by the controller 14 in response to sensed movement of the second phalange of the surgeon's right finger. As with the other portions of controlled hand 12, movement of the left or right second phalange 76, 78 is in "one-for-one" correspondence with that of the second phalange of the surgeon's left or right finger, as defined hereinabove.

The construction of the parts of the controlled hand providing finger spreading is as follows. The first motion transmission rod 50 is connected at its distal end to a stiffly flexible cable 50a. A distal end of the cable 50a is secured to spreader links 86, 88 which are pivotally connected to each other and to the controlled fingers, as described hereinbefore. The attachment to each controlled finger is made at left and right spreader link attachment pints, 86a and 88a, respectively, on a first phalange 72 and 74 of each controlled finger. These two links 86 and 88 are movably connected at both ends, to provide swiveling in response to movement of the first rod 50.

When the first rod 50 and its attached cable 50a move distally due to actuation of the servomotor 50M by the microprocessor provided by the controller 14 in response to sensed relative movement between the surgeon's left and right fingers, each link is caused to move distally. Since the link's distal end is attached to the controlled fingers, the distal end of the links 86, 88 cannot move distally, but instead moves outward, causing the fingers to swivel outwardly away from each other. Conversely, when the first rod 50 and its attached cable 50a move proximally, the links 86, 88 are drawn proximally, causing the controlled fingers to swivel back together.

The controlled fingers spread in response to movement of the first motion transmission rod 50 and the cable 50a. When the surgeon's fingers are moved relative to one another from an aligned position to a V-shaped position, the sensors provided by the data glove or exoskeleton 22 detect the relative movement or the change in spacing between the fingers and transmit an electrical signal representing the sensed position of the surgeon's fingers through the data link 20 to the controller 14. In response to the change in the sensed position of the surgeon's fingers, the controller actuates the servomotor 50M and moves the first motion transmission rod 50 and cable 50a longitudinally in the distal direction, i.e., relatively toward the controlled hand 12. When the rod 50 and cable 50a moves toward the controlled hand 12 the controlled fingers are caused to spread or move laterally away from each other.

Conversely, when the surgeon's fingers are moved toward each other, the servomotor 50M is reversely actuated, and moves the first motion transmission rod 50 and cable 50a relatively away from the controlled hand 12. The controlled fingers move back toward each other when the first rod 50 is drawn back away from the controlled hand 10. Thus, the sensed position of the surgeon's fingers is used to actuate or control the servomotor 50M which moves the first motion transmission rod 50 and cables 50a, and creates a one-for-one correspondence between the positions of the surgeon's fingers and the controlled fingers.

In the embodiment thus described, the finger spreading and returning capability creates an equal and opposite motion of finger assemblies of the controlled hand 12, to be described more fully hereafter. In other words if, for example, the right finger assembly (i.e., first and second phalanges 72, 76 of the right finger) spreads outward at an angle of 25°, the mechanism as shown in the drawings operates such that left finger assembly (i.e., first and second phalanges 72, 76 of the left finger) simultaneously spreads 25° outward, in the same plane but in a direction laterally opposite that in which right finger assembly moves. While this is the preferred embodiment, other embodiments are possible wherein the left and right finger assemblies are independently spreadable, or in which the simultaneous spreading is not equal in both directions.

The thumb portion of the controlled hand 12 is mounted on the controlled thumb pivot base posts 66. The controlled thumb includes a first phalange 100 which has a second phalange 102 pivotally mounted thereto. The controlled thumb second phalange 102 is substantially similar to and operates according to the same principles as does the controlled finger second phalanges 76, 78, described hereinbefore.

The thumb first phalange 100 includes a pair of parallel, interconnected flat links 100a and 100b. Thumb base mount openings 104 are provided in each link 100a and 100b, and permit pivotal mounting of the firs phalange 100 on the thumb pivot base posts 66 of the pivot post 36, and allow for movement of the controlled thumb in a vertically oriented plane (i.e., about a generally horizontal axis). Relative to the controlled fingers, movement of the controlled thumb is equivalent to palmar adduction and abduction, wherein the thumb as a unit moves toward or away from the fingers of the human hand.

An adduction cable mount or opening 106 provides an attachment point for a link 53b which is actuated by stiffly flexible cable 53a fixed to the end of the fourth motion transmission rod 53. Movement of the cable 53a and rod 53 causes the link 53b to actuate thumb first phalange 100, and forces the thumb first phalange 100 to pivot about its attachment at the base mount openings 104, thus creating the movement of the controlled thumb corresponding to palmar adduction and abduction. The link 53b is attached to the controlled thumb first phalange 100 by a pivot pin 106a. The motion transmitting rod 53 is actuated and controlled by its corresponding servomotor 53M which is operated by the controller 14 in response to sensed movement of the surgeon's thumb first phalange. As with other parts of controlled hand 12, the adduction and abduction movements of the controlled thumb are in one-for-one correspondence, as defined, with similar movements of the surgeon's thumb.

A distal end of the controlled thumb first phalange 100 is further provided with a second phalange mount or opening 174 in each link 100a, 100b for a pair of mounting posts 110 of the thumb second phalange 102. The point of attachment is preferably referred to as an interphalangeal joint hinge. The interphalangeal joint hinge at which the first and second phalanges 100, 102 are attached to one another corresponds to an interphalangeal joint on the surgeon's thumb.

The thumb second phalange 102 may be made from round stock, with a flattened segment extending part of the length of the second phalange, preferably at least to the vicinity of a flexion cable mount opening 112, as illustrated. A distal portion 114 of the thumb second phalange 102 is preferably cylindrical, with a rounded terminal end. The distal portion 114 may be made of the same or different material as the remainder of the thumb second phalange 102, which in turn may be formed of material the same as or different from that of the controlled hand 18.

The controlled thumb second phalange 102 is actuated and controlled by a stiffly flexible cable 54a connected to the distal end of the fifth motion transmission rod 54 which is, in turn, actuated and controlled by its associated servomotor 54M. The servomotor 54M is operated by the microprocessor provided by the controller 14 in response to sensed movement of the surgeon's thumb second phalange. As with the other portions of controlled hand 12, movement of the controlled thumb second phalange 102 is in one-for-one correspondence with that of the surgeon's thumb second phalange, as defined above.

Each of the above described elements of the controlled hand is independently responsive to movements of the particular motion transmission rod, and associated flexible cables, with which that element has been described. Thus, each of these six motion transmission rods 50, 51, 52, 53, 54, 55, operating the fingers, the thumb and the wrist 56, is separately operable by their corresponding servomotors 50M, 51M, 52M, 53M, 54M, 55M and the microprocessor provided by the controller 14 as a result of sensed movement of the corresponding portions of the surgeon's hand.

Thus, the six motion transmission means comprising the rods and cables perform the following functions: The first rod 50 and its cable 50a connect and provide spreading movement to the controlled fingers, via the spreader links 36, 88. The second rod 51 and its cable 5la transmit movement to the left controlled finger second phalange 76. The third rod 52 and its cable 52a transmit movement to the right controlled finger second phalange 78. The fourth rod 53 and its cable 53a provide the controlled thumb with the capability of palmar adduction and abduction, by connecting the controller 14 to the first phalange 100 of the controlled thumb. The fifth rod 54 and its cable 54a connect the control means to the controlled thumb second phalange 102. The sixth rod 55 and its cable 55a provide actuating connection between the controller 14 and the controlled wrist 56. Thus, the second, third, and fifth rods 51, 52, 54 provide the fingers and thumb of the controlled hand 10 with the capability of moving in flexion and extension.

It should be apparent that the invention described herein is capable of numerous modifications, substitutions, and rearrangements of parts without departing from the scope and spirit of the present invention. Therefore, the present invention is not to be limited to the specific structure described herein, but rather is only defined by the claims and their equivalents appended hereto.

## Claims

1. A system for use in endoscopic surgery, comprising:
a surgical tool having a plurality of gripping members which are movable relative to one another to perform a surgical procedure, said tool being adapted for insertion through a cannula to an internal surgical site;
means for visualizing the internal surgical site;
means for controlling movement of the gripping members;
means for operatively linking the control means to the tool and thereby allowing the control means to adjust the position of any one gripping member relative to the other gripping members;
means for communicating the sensed position of the surgeon's fingers to the controlling means, whereby sid controlling means, via said linking means, adjusts said gripping members such that a relative position of said gripping members is proportional to the sensed relative position of sid surgeon's fingers, thereby allowing the surgeon to remotely perform a desired surgical movement.

2. A system for use in laparoscopy surgery according to claim 1, wherein said visualizing means comprises a laparoscopy camera and light source, said camera and light source providing illumination and remote visualization of the internal surgical site.

3. A system for use in laparoscopy surgery according to claim 2, wherein sid visualizing mens further comprises a video display device.

4. A system for use in laparoscopy surgery according to claim 2, wherein said video display device is in a location remote from a patient being operated upon.

5. A system for use in laparoscopy surgery according to claim 1, wherein said sensing means comprises a data glove which is worn by the surgeon.

6. A system for use in laparoscopy surgery according to claim 1, wherein said sensing means comprises an exoskeleton which is worn by the surgeon.

7. A system for use in laparoscopy surgery according to claim 1, wherein the controlling means includes a series of servomotors and a microprocessor, said microprocessor receiving signals representative of the sensed relative position of the surgeon's fingers and actuating one or more servomotors which, via said linking means, moves the gripping members to a position corresponding to the sensed relative position of the surgeon's fingers.

8. A system for use in laparoscopy surgery according to claim 7, wherein the linking means comprises a series of control rods and associated control wires, said control rods and wires operatively connecting the servomotors to the gripping members.

9. A system for use in laparoscopy surgery according to claim 8, wherein the surgical procedure is microsurgery and movement of the gripping members is a fraction of sensed movement of the surgeon's fingers.

10. A system for use in endoscopic surgery, comprising:
a surgical tool having a plurality of gripping members which are movable relative to one another to perform a surgical procedure, said tool being adapted for insertion through a cannula to an internal surgical site;
means for remotely visualizing the surgical site;
a controller which controls movement of the gripping members;
a connecting assembly for mechanically linking the controller to the surgical tool, said connecting assembly allowing the controller to adjust the position of any one gripping member relative to the other gripping members;
a sensing device for sensing a relative position of a plurality of a surgeon's fingers; and,
a data link for communicating the sensed relative position of the surgeon's fingers to the controller, whereby said controller, via said connecting assembly, moves said gripping members to maintain the relative positions of the gripping members proportional to the sensed relative position of the surgeon's fingers and thereby allow the surgeon to remotely perform a des-ired surgical movement.

11. A system according to claim 10, wherein each of said plurality of gripping members includes first and second phalanges which are connected by an interphalangeal joint, each second phalange being movable independently of the associated first phalange.

12. A system according to claim 11, wherein said controller comprises a series of servomotors and a microprocessor, said microprocessor receiving signals of the sensed relative position of the surgeon's fingers and operating said servomotors such that said gripping members are positioned proportionally identical to said sensed relative position of the surgeon's fingers.

13. A system according to claim 12, wherein said connecting assembly comprises a series of control rods, said control rods operatively connecting the phalanges with an associated servomotor.

14. A system according to claim 13., wherein the surgical procedure is microsurgery and movement of the gripping members is a predetermined fraction of a sensed relative movement of the surgeon's fingers.
